# EUROPÄISCHE PATENTSCHRIFT

(11) **EP 2 183 208 B1**
(45) Veröffentlichungstag und Bekanntmachung des Hinweises auf die Patenterteilung: **25.01.2017**
(21) Anmeldenummer: 08775077.4
(22) Anmeldetag: 15.07.2008
(51) Int. Cl.: C07C 37/74, C07C 37/68, C07C 37/84

(54) **DIALKYLPHENOLE**
DIALKYL PHENOLS
DIALKYLPHÉNOLS

(30) Priorität: 28.07.2007 DE 102007035515
(43) Veröffentlichungstag der Anmeldung: 12.05.2010
(73) Patentinhaber: LANXESS Deutschland GmbH, 50569 Köln (DE)
(72) Erfinder: HEUER, Lutz, 41542 Dormagen (DE)
(86) Internationale Anmeldenummer: PCT/EP2008/059217
(87) Internationale Veröffentlichungsnummer: WO 2009/016028

(56) Entgegenhaltungen:
- GB-A- 285 833
- GB-A- 1 227 924
- US-A- 1 886 311
- US-A- 2 468 670
- US-A- 3 992 455

## Beschreibung

Die vorliegende Erfindung betrifft ein Verfahren zur Herstellung von 4-Isopropyl-3-methyl-phettol durch Alkylierung von meta-Kresol, Destillation und Kristallisation.

4-Isopropyl-3-methyl-phenol wird beispielsweise als antibakterielles und mikrobizides Mittel in Kosmetika, Mundwässern und Shampoos mit guter Hautverträglichkeit eingesetzt. Die Herstellung von 4-Isopropyl-3-methyl-phenol ist prinzipiell bekannt.

So beschreibt US 3,331,879 die Umsetzung von meta-Kresol (m-Kresol, 3-Methylphenol) mit Propen an einem Zirkoniumkatalysator, wobei im Wesentlichen Thymol (2-Isopropyl-5-methyl-phenol), aber auch viele aromatische Nebenprodukte entstehen. Als ein Nebenprodukt wurde 4-Isopropyl-3-methyl-phenol mit einem Gehalt von 2 % in der Reaktionsmischung bzw. 4,4 % nach einer ersten Destillation identifiziert. Die Isolierung des 4-Isopropyl-3-methyl-phenols wird nicht beschrieben.

DE 2139622 OS beschreibt den Anfall von bis zu 19,5 % 4-Isopropyl-3-methyl-phenol bei der Umsetzung von m-Kresol mit Propen an einem sauren Zinkkatalysator. Auch hier wird die Isolierung des 4-Isopropyl-3-methyl-phenols nicht beschrieben.

DE 2528303 OS beschreibt den Anfall von ca. 2 % 4-Isopropyl-3-methyl-phenol bei der Umsetzung von meta-Kresol mit Propen an einem basischen Aluminiumoxid-Katalysator. Isolierung des 4-Isopropyl-3-methyl-phenols aus den vielfältigen Nebenprodukten wird nicht beschrieben.

Weiterhin ist aus US 2,603,662 bekannt 4-Isopropyl-3-methyl-phenol über einen aufwändigen Prozess als Nebenprodukt bei der Umsetzung von meta-Kresol mit Isobuten zu gewinnen.

US 1886311 offenbart ein Verfahren zur Herstellung und Kristallisation von 4-Isopropyl-3-methyl-phenol.

Allen vorgenannten Verfahren ist gemeinsam, dass 4-Isopropyl-3-methyl-phenol als Nebenkomponente bei der Alkylierung von m-Kresol mit so vielen anderen Nebenkomponenten anfällt, dass eine Gewinnung entweder nicht vorgenommen wurde oder außerordentlich umständlich erfolgt. Es bestand daher das Bedürfnis, ein Verfahren bereitzustellen, mit 4-Isopropyl-3-methyl-phenol in effizienter Weise gewonnen werden kann.

Es wurde nun ein Verfahren zur Herstellung von 4-Isopropyl-3-methyl-phenol durch Umsetzung von meta-Kresol mit Propen gefunden, das dadurch gekennzeichnet ist, dass
a) Thymol und nicht-umgesetztes meta-Kresol destillativ aus der Reaktionsmischung weitgehend abgetrennt werden und
b) der aus Schritt a) verbleibende Rückstand dann destilliert wird um schwerst- oder nichtflüchtige Substanzen abzutrennen und das so erhaltene Destillat nach Zusatz von bis zu 5 Gew.-% Wasser kristallisiert wird oder
c) der aus Schritt a) verbleibende Rückstand, nach Zusatz von bis zu 5 Gew.-% Wasser, kristallisiert wird und der kristallisierte Rückstand durch Destillation von schwerst- oder nichtflüchtigen Substanzen getrennt wird.

Der Rahmen der Erfindung umfasst neben den genannten Bereichen und Vorzugsbereichen von Formeln und Parametern auch beliebige Kombinationen davon, selbst wenn sie aus praktischen Gründen nachstehend nicht vollständig explizit aufgeführt sind.

Bei der Alkylierung von meta-Kresol mit Propen, die in einer dem Fachmann bekannten Art und Weise durchgeführt werden kann (siehe z.b. DE 3824284 OS oder DE 2528303 OS) entstehen typischerweise ein Reaktionsgemisch, das etwa 1 bis 3 Gew.-% 4-Isopropyl-3-methylphenol enthält.

In einem Schritt a) des erfindungsgemäßen Verfahrens werden Thymol und nicht-umgesetztes meta-Kresol aus der Reaktionsmischung destillativ weitgehend abgetrennt. Der Begriff weitgehend bedeutet dabei, dass der verbleibende Rückstand einen Anteil an Thymol und meta-Kresol von zusammengenommen insgesamt 80 % oder weniger, bevorzugt 55 % oder weniger und besonders bevorzugt 20 % oder weniger aufweist.

Die Destillation kann dabei in an sich bekannter Weise beispielsweise diskontinuierlich oder kontinuierlich durchgeführt werden, wobei eine kontinuierliche Destillation unter gegenüber Normaldruck vermindertem Druck bevorzugt ist.

Typischerweise enthält der verbleibende Rückstand neben 4-Isopropyl-3-methylphenol 20 bis 30 weiteren Nebenkomponenten niedermolekularer Struktur sowie polymere Nebenkomponenten. Nach der Destillation beträgt der Gehalt von 4-Isopropyl-3-methylphenol im verbleibenden üblicherweise schwarz gefärbten Rückstand typischerweise 10 bis 30 Gew.-%

Gemäß Schritt b) kann der aus Schritt a) verbleibende Rückstand zunächst destilliert werden, um schwerst- oder nichtflüchtige Substanzen abzutrennen und das so erhaltene Destillat nach Zusatz von bis zu 5 Gew.-% Wasser kristallisiert werden.

Alternativ dazu kann gemäß Schritt c) der aus Schritt a) verbleibende Rückstand kristallisiert werden und der kristallisierte Rückstand durch Destillation von schwerst- oder nichtflüchtigen Substanzen getrennt werden.

Die Destillation in Schritt b) oder c) kann dabei in an sich bekannter Weise beispielsweise kontinuierlich oder diskontinuierlich erfolgen. Vorzugsweise erfolgt die Destillation mit Hilfe eines Kurzwegverdampfers, einer Kolonne ohne Einbauten oder durch einen Fallfilmverdampfer oder auch einen Dünnschichtverdampfer. Für die Destillation ist eine theoretische Trennstufe ausreichend.

Geeignete Destillationsbedingungen sind für den Fachmann ohne Weiteres ermittelbar.

In einer Ausführungsform des erfindungsgemäßen Verfahrens können die Destillationen der Schritte a) und b) auch simultan durchgerührt werden. In diesem Fall ist die Destillation in einer Seitenabzugskolonne bevorzugt.

Gemäß Schritt b) wird das Destillat, gemäß Schritt c) der aus Schritt a) verbleibende Rückstand kristallisiert. Die Kristallisation kann dabei beispielsweise bei Temperaturen von -50 bis 80 °C erfolgen. Die Kristallisation erfolgt vorzugsweise bei Temperaturen von 0 bis 50°C besonders bevorzugt bei Temperaturen von 5 bis 35°C.

In einer Ausführungsform der Erfindung wird das Destillat gemäß Schritt b) oder der aus Schritt a) verbleibende Rückstand mit Wasser versetzt, vorzugsweise mit 0,01 bis 5 Gew.-% bezogen auf das Gewicht des Destillats besonders bevorzugt 1 bis 5 Gew.-%.

In einer weiteren Ausführungsform erfolgt die Kristallisation derart, dass das Destillat gemäß Schritt b) oder der aus Schritt a) verbleibende Rückstand mit kristallinem 4-Isopropyl-3-methylphenol versetzt wird, vorzugsweise mit wenigen Kristallen. Die Kristallisation kann dabei kontinuierlich oder diskontinuierlich erfolgen. Beim Stehenlassen, vorzugsweise für 2 bis 200 Stunden, bildet sich ein Kristallkuchen, aus dem durch Dekantieren 4-Isopropyl-3-methylphenol gewonnen werden kann, das typischerweise eine Reinheit von 85 bis 95 % bezogen auf das Gewicht aufweist. Überraschenderweise kann die Kristallisation ohne Lösungs- oder Verdünnungsmittel erfolgen, obgleich auch die andere Substanzen des Rückstandes aus Schritt a) bzw. des Destillates gemäß Schritt b) Feststoffe sind. Beispiele dafür sind 2-Isopropyl-5-methylphenol mit einem Schmelzpunkt von 49 °C , 3-Methylphenol mit einem Schmelzpunkt von 12 °C und 3-Isopropyl-5-methylphenol Schmelzpunkt mit einem Schmelzpunkt von 49 bis 51 °C.

Das gemäß Schritt b) oder c) gewonnene, kristalline 4-Isopropyl-3-methylphenol kann durch Waschen mit einem C₁ bis C₄-Alkohol, Mischungen solcher Alkohole oder Mischungen von einem oder mehreren dieser Alkohole mit Wasser weiter gereinigt werden. Bevorzugte C₁ bis C₄-Alkohole sind Methanol, Ethanol und Isopropanol. Bei Mischungen mit Wasser beträgt der Wasseranteil beispielsweise 1 bis 90 Gew.-% bevorzugt 40 bis 90 Gew.-%.

Zur weiteren Erhöhung der Reinheit kann das erfindungsgemäß erhältliche 4-Isopropyl-3-methylphenol auch umkristallisiert werden. Bevorzugte Lösungsmittel für die Umkristallisation sind die vorstehend genannten C₁ bis C₄-Alkohole, Mischungen solcher Alkohole oder Mischungen von einem oder mehreren dieser Alkohole mit Wasser. Bevorzugt erfolgt die Umkristallisation unter Hinzufügen von Filtrationshilfsmitteln oder Aktivkohle.

In einer weiteren Ausführungsform kann die Kristallisation derart erfolgen, dass die Kristallisation kontinuierlich oder diskontinuierlich durch Abkühlen erfolgt. Vorzugsweise erfolgt das Abkühlen an einem im Vergleich zur Umgebungstemperatur gekühlten Rohr, Rohrbündel oder durch sonstige Einbauten kühlbaren Behälter, wobei die entstandenen Kristalle nach Ablaufen der anderen Verbindungen durch Erwärmen des Rohres, Rohrbündels oder der sonstigen durch Einbauten kühlbaren Behälter abgeschmolzen werden. Vorzugsweise wird dieser Prozess wiederholt, wobei sehr reines 4-Isopropyl-3-methylphenol gewonnen wird.

Bei der Verfährensführung gemäß der Schritte a) und c) ist vorteilhaft, dass bei gleichen Operationen sehr reines 4-Isopropyl-3-methylphenol erhalten werden kann.

Der besondere Vorteil der Erfindung ist darin zu sehen, dass 4-Isopropyl-3-methyl-phenol als Nebenkomponente bei der Thymol-Herstellung trotz Vorliegen sehr vieler weiterer, bei Raumtemperatur ebenfalls festen Nebenkomponenten, effizient und in hoher Reinheit gewonnen werden kann.

### Beispiel:

Bei 150°C und 1 mBar wurden 1000 g schwarzer Rückstand aus der Thymol-Herstellung, enthaltend 18,5 Gew.-% Thymol, 9,5 Gew.-% 3-Isopropyl-5-methyl-phenol, 26,2 Gew.-% 4-Isopropyl-3-methyl-phenol, 16,5 Gew.-% 2,6-Diisopropyl-3-methyl-phenol, 24,2 Gew.-% 2,4-Diisopropyl-5-methyl-phenol sowie etwa 22 verschiedene in Summe ca. 5 Gew.-% sonstiger alkylierter Kresole unter Verwendung eines Claissen-Aufsatzes ohne Kolonne destilliert. Es wurden 993,3 g eines gelblichen Destillates erhalten, das in etwa die vorgenannte Zusammensetzung aufwies. 200 g dieses Öls wurden bei 22 °C mit 0,4 g reinem, kristallinen 4-Isopropyl-3-methyl-phenol, suspendiert in 3 ml Wasser, angeimpft. Nach 48 h wurden 40,2 g (76,7 % d.Th.) farblose Kristalle von 4-Isopropyl-3-methyl-phenol erhalten, die nach Waschen mit etwas Methanol eine Reinheit von 95,1 % aufwiesen.

## Patentansprüche

1. Verfahren zur Herstellung von 4-Isopropyl-3-methyl-phenol durch Umsetzung von meta-Kresol mit Propen, **dadurch gekennzeichnet, dass**
a) Thymol und nicht-umgesetztes meta-Kresol destillativ aus der Reaktionsmischung weitgehend abgetrennt werden und
b) der aus Schritt a) verbleibende Rückstand dann destilliert wird um schwerst- oder nichtflüchtige Substanzen abzutrennen und das so erhaltene Destillat nach Zusatz von bis zu 5 Gew.-% Wasser kristallisiert wird oder
c) der aus Schritt a) verbleibende Rückstand, nach Zusatz von bis zu 5 Gew.-% Wasser, kristallisiert wird und der kristallisierte Rückstand durch Destillation von schwerst- oder nichtflüchtigen Substanzen getrennt wird.

2. Verfahren nach Anspruch 1, **dadurch gekennzeichnet, dass** die Destillationen der Schritte a) und b) simultan durchgeführt werden

3. Verfahren nach Anspruch 1 oder 2, **dadurch gekennzeichnet, dass** die Kristallisation gemäß Schritt b) oder c) bei Temperaturen von -50 bis 80 °C erfolgt.

4. Verfahren nach einem der Ansprüche 1 bis 3, **dadurch gekennzeichnet, dass** das Destillat aus Schritt b) oder der Rückstand aus Schritt a) mit Wasser versetzt wird.

5. Verfahren nach einem der Ansprüche 1 bis 4, **dadurch gekennzeichnet, dass** das Destillat aus Schritt b) oder der Rückstand aus Schritt a) mit kristallinem 4-Isopropyl-3-methylphenol versetzt wird.

6. Verfahren nach einem der Ansprüche 1 bis 5, **dadurch gekennzeichnet, dass** die Kristallisation durch Abkühlen erfolgt.

7. Verfahren nach Anspruch 6, **dadurch gekennzeichnet, dass** das Abkühlen an einem im Vergleich zur Umgebungstemperatur gekühlten Rohr, Rohrbündel oder durch sonstige Einbauten kühlbaren Behälter erfolgt, wobei die entstandenen Kristalle nach Ablaufen der anderen Verbindungen durch Erwärmen des Rohres, Rohrbündels oder des durch sonstige Einbauten kühlbaren Behälters abgeschmolzen werden.

## Claims

1. Process for preparing 4-isopropyl-3-methylphenol by reacting meta-cresol with propene, **characterized in that**
a) thymol and unreacted meta-cresol are largely removed distillatively from the reaction mixture and
b) the residue remaining from step a) is then distilled in order to remove extremely unvolatile or nonvolatile substances and the resulting distillate, after addition of up to 5% by weight of water, is crystallized, or
c) the residue remaining from step a), after addition of up to 5% by weight of water, is crystallized and the crystallized residue is separated by distillation from extremely unvolatile or nonvolatile substances.

2. Process according to Claim 1, **characterized in that** the distillation of steps a) and b) are carried out simultaneously.

3. Process according to Claim 1 or 2, **characterized in that** the crystallization as per step b) or c) takes place at temperatures of -50 to 80°C.

4. Process according to any of Claims 1 to 3, **characterized in that** the distillate from step b) or the residue from step a) is admixed with water.

5. Process according to any of Claims 1 to 4, **characterized in that** the distillate from step b) or the residue from step a) is admixed with crystalline 4-isopropyl-3-methylphenol.

6. Process according to any of Claims 1 to 5, **characterized in that** the crystallization takes place as a result of cooling.

7. Process according to Claim 6, **characterized in that** the cooling takes place in a tube, tube bundle or container coolable by means of other internals, this apparatus being cooled in comparison to the ambient temperature, and the resultant crystals, after the other compounds have been drained off, are melted off by heating of the tube, tube bundle or container coolable by other internals.

## Revendications

1. Procédé pour la préparation de 4-isopropyl-3-méthylphénol par transformation de méta-crésol avec du propène, **caractérisé en ce que**
a) du thymol et du méta-crésol non transformé sont éliminées, dans une large mesure, par distillation du mélange réactionnel et
b) le résidu qui reste de l'étape a) est ensuite distillé pour séparer les substances peu volatiles ou non volatiles et le distillat ainsi obtenu est cristallisé après addition de jusqu'à 5% en poids d'eau ou
c) le résidu qui reste de l'étape a) est cristallisé après addition de jusqu'à 5% en poids d'eau et le résidu cristallisé est séparé par distillation des substances peu volatiles ou non volatiles.

2. Procédé selon la revendication 1, **caractérisé en ce que** les distillations des étapes a) et b) sont réalisées simultanément.

3. Procédé selon la revendication 1 ou 2, **caractérisé en ce que** la cristallisation selon l'étape b) ou c) a lieu à des températures de -50°C à 80°C.

4. Procédé selon l'une quelconque des revendications 1 à 3, **caractérisé en ce que** le distillat de l'étape b) ou le résidu de l'étape a) est additionné d'eau.

5. Procédé selon l'une quelconque des revendications 1 à 4, **caractérisé en ce que** le distillat de l'étape b) ou le résidu de l'étape a) est additionné de 4-isopropyl-3-méthylphénol cristallin.

6. Procédé selon l'une quelconque des revendications 1 à 5, **caractérisé en ce que** la cristallisation a lieu par refroidissement.

7. Procédé selon la revendication 6, **caractérisé en ce que** le refroidissement a lieu au niveau d'un tube, d'un faisceau tubulaire ou d'un récipient pouvant être refroidi par d'autres inserts, refroidi par rapport à la température ambiante, les cristaux formés étant fondus après l'évacuation des autres composés par réchauffement du tube, du faisceau tubulaire ou du récipient pouvant être refroidi par d'autres inserts.
